# EUROPÄISCHE PATENTSCHRIFT

(11) **EP 0 601 339 B1**
(45) Veröffentlichungstag und Bekanntmachung des Hinweises auf die Patenterteilung: **06.05.1999**
(21) Anmeldenummer: 93118003.8
(22) Anmeldetag: 05.11.1993
(51) Int. Cl.: A61N 1/05

(54) **Defibrillierungselektrode**
Defibrillation electrode
Electrode de défibrillation

(30) Priorität: 11.12.1992 SE 9203733
(43) Veröffentlichungstag der Anmeldung: 15.06.1994
(73) Patentinhaber: Pacesetter AB, 171 95 Solna (SE)
(72) Erfinder: Hirschberg, Jakub, S-183 44 Täby (SE); Bowald, Staffan, S-740 10 Almunge (SE)

(56) Entgegenhaltungen:
- WO-A-92/11898
- US-A- 4 503 569
- US-A- 4 573 481
- US-A- 4 825 871
- US-A- 4 860 769
- US-A- 4 920 979

## Beschreibung

Die Erfindung betrifft eine Defibrillierungselektrode, insbesondere für die intravaskulare Plazierung in einem Patienten, mit einem flexiblen Elektrodenkabel, das mindestens einen langgestreckten elektrisch isolierten Leiter umfasst und mit einem am distalen Ende des Elektrodenkabels angebrachten Elektrodenkopf, der mindestens eine Defibrillierungsoberfläche zur Übertragung von Defibrillierungsimpulsen an das Herz des Patienten aufweist, gemäss den ersten Teil des Anspruches 1.

Eine solche Defibrillierungselektrode, die für die intravaskulare Plazierung in einem Patienten vorgesehen ist, ist durch die WO-A-92/11898 bekannt.

Der allgemeine Stand der Technik wird auch durch den folgenden Patentschriften illustriert.

EP-A 0 373 953: Der Elektrodenkopf der hier beschriebenen Defibrillierungselektrode ist eine Verlängerung des Elektrodenkabels, so dass der Durchmesser des Elektrodenkopfes etwa dem Durchmesser des Elektrodenkabels entspricht. Ein Nachteil dieser Defibrillierungselektrode ist, dass der Elektrodenkopf bei einer Applizierung in einem Blutgefäss nicht fixierbar ist. Da eine Defibrillierungselektrode dieser Art immer ein Teil eines Defibrillierungssystems mit mehreren Defibrillierungselektroden ist, kann eine Verschiebung des Elektrodenkopfes in der Längsrichtung eine unerwünschte Stromverteilung im Herz mitsichführen. Ein weiterer Nachteil ist, dass der Elektrodenkopf in der Blutbahn des Gefässes ohne eine seitliche Steuerung angebracht ist. Ein solcher Elektrodenkopf kann den Blutfluss verhindern gleichzeitig damit, dass die Gefahr besteht, dass Blutklumpen gebildet werden können.
In der US-PS 4 860 769 ist eine Defibrillierungselektrode von einer sog. Patch-Art beschrieben, deren Elektrodenkopf aus einem wendelförmigen Leiter derart aufgebaut ist, dass der Elektrodenkopf im Profil gesehen konusformig ist. Diese Defibrillierungselektrode ist dazu vorgesehen, im Epikardium appliziert zu werden und eignet sich nicht für eine intravaskulare Plazierung.

Aus der US-PS 4 414 986 ist ein Elektrodenkabel mit einem am Kabel angebrachten Elektrodenring bekannt, der für die Stimulation des Rückenmarks eines Patienten vorgesehen ist. Das Elektrodenkabel, das gegen die harte Haut des Rückenmarks anliegen soll, ist teilweise wendelförmig vorgeformt, damit das Kabel in einer gewünschten Lage fixiert werden kann. Das distale Ende des Elektrodenkabels ist mit weiteren Fixiermitteln in Form von nach aussen ragenden Teilen versehen. Ein derartiges Elektrodenkabel eignet sich nicht für eine intravaskulare Applizierung, da die herausragenden Teile die umgebende Blutgefässwand mit grosser Wahrscheinlichkeit beschädigen würden. Ausserdem würde aufgrund der Plazierung und der Form der herausragenden Teile das Kabel etwa im Zentrum des Blutgefässes zu liegen kommen, was den Blutfluss verhindern würde.

Der Erfindung liegt die Aufgabe zugrunde, eine Defibrillierungselektrode der eingangs genannten Art zu schaffen, die fixierbar ist und die den Blutfluss in demjenigen Blutgefäss, in dem die Elektrode fixiert ist, geringfügig beeinflusst und die auch nicht bei einer hohen Stromabgabe die umgebende Gefässwand beschädigt.

Diese Aufgabe ist erfindungsgemäss dadurch gelöst, dass der Elektrodenkopf derart aufgebaut ist, dass er in radialer Richtung expandierbar ist und in einer expandierten Lage wenigstens die Konturen eines Hohlkörpers bildet, wobei die nach innen gerichtete Wand des gesamten Hohlkörpers die Defibrillierungsoberfläche bildet. Auf diese Weise wird eine verhältnismässig grosse Defibrillierungsoberfläche erhalten, die auch nicht bei einer hohen Stromabgabe die umgebende Gefässwand beschädigt.

Der Elektrodenkopf kann in einer nicht expandierten Lage einen Durchmesser aufweisen, der etwa dem Durchmesser des Elektrodenkabels entspricht, und bildet eine Verlängerung des Elektrodenkabels in dessen Längsrichtung. Auf diese Weise kann das Elektrodenkabel mit dem Elektrodenkopf ohne Schwierigkeiten in ein Blutgefäss eingeführt werden. In einer expandierten Lage überschreitet der Radius des Hohlkörpers den Radius des Blutgefässes derart, dass der Hohlkörper bei einer Applizierung gegen die umgebende Gefässwand druckfixiert wird. Dadurch, daß der Elektrodenkopf hohl ist und gegen die Gefässwand anliegt, wird der Blutfluss geringfügig beeinflusst.

In einer vorteilhaften Ausbildung der Erfindung wird vorgeschlagen, dass der Hohlkörper aus einem schraubenförmig gewickelten Leiter besteht. Der Vorteil einer solchen Konfiguration ist, dass sie sehr flexibel ist. Dies hat zur Folge, dass der Hohlkörper auch in einem verhältnismässig kraftig gebogenen Teil eines Blutgefässes applizierbar ist.

In einer Weiterbildung der Erfindung wird vorgeschlagen, dass der Hohlkörper teils aus Umdrehungen eines schraubenförmig gewickelten Leiters, der in diesen Abschnitten elektrisch isoliert ist, und teils aus einem nicht schraubenförmig gewickelten, hauptsächlich geraden, nicht isolierten Leiter besteht. Bei einer solchen Ausführungsform des Elektrodenkopfes kann die leitende Oberfläche, in eine gewünschte Richtung gerichtet werden, in dem der Hohlkörper um seine Längsachse gedreht wird. Auf diese Weise kann durch die Elektrodenvorrichtung eine günstige Stromverteilung im Herzgewebe erhalten werden.

Im Hinblick auf eine weitere Ausgestaltung der Erfindung wird vorgeschlagen, dass der Leiter des Elektrodenkabels und der schraubenförmig gewickelte Leiter einen Kanal aufweisen, der sich über die gesamte Länge der Defibrillierungselektrode erstreckt und der zum Einführen eines Mandrins vorgesehen ist. Hierdurch wird erreicht, dass der Elektrodenkopf bzw. der Hohlkörper mit Hilfe des Mandrins ausgerichtet wird, so dass der Durchmesser des Hohlkörpers etwa dem Durchmesser des Elektrodenkabels entspricht. Dies bedeutet, dass der Durchmesser des Hohlkörpers nun auch kleiner als der Innendurchmesser derjenigen Blutgefässe ist, die passiert werden sollen und kleiner als der Innendurchmesser des Blutgefässes, in dem der Hohlkörper appliziert werden soll. Wenn der Arzt, der die Elektrodenvorrichtung implantiert, eine günstige Lage im Blutgefäss gefunden hat, kann der Mandrin herausgezogen werden, wobei die Elektrodenvorrichtung wieder ihre ursprüngliche Form einnimmt.

Eine konstruktiv einfache Ausführung der Erfindung ist dadurch gegeben, dass der Elektrodenkopf aus einer Folie besteht, die in einer nicht expandierten Lage spiralenförmig zusammengerollt ist. Wenn der Elektrodenkopf eine expandierte Lage einnimmt, wird eine ausserordentlich grosse Defibrillierungsoberfläche erhalten.

In einer vorteilhaften Ausgestaltung der Erfindung wird vorgeschlagen, dass der Hohlkörper die Form eines Zylinders aufweist.

In einer weiteren vorteilhaften Ausbildung der Erfindung wird vorgeschlagen, dass der Zylinder aus parallel zueinander verlaufenden drahtförmigen Leitern besteht, die entlang der Längsachse des Zylinders verlaufen und die mittels zusammendrückbarer Verbindungselemente miteinander verbunden sind.

In einer weiteren im Aufbau einfachen Ausführungsform der Erfindung wird vorgeschlagen, dass der Zylinder aus mindestens einem drahtförmigen Leiter besteht, der zickzackförmig entlang der Längsachse des Zylinders verläuft. Durch diesen konstruktiven Aufbau ist es möglich, den Zylinder in verschiedenen Blutgefässen mit den unterschiedlichsten Innendurchmessern anzubringen ohne die Gefahr, dass ein Teil des Zylinders in die Blutbahn ragt und den Blutfluss hindert.

Der Elektrodenkopf kann nach der Erfindung in einer nicht expandierten Lage in einem Einführungskatheter appliziert werden. Hierdurch ist erreicht, dass das Elektrodenkabel mit dem Elektrodenkopf in ein Blutgefäss eingeführt werden kann, ohne die umgebende Gefässwand zu beschädigen.

Der Elektrodenkopf kann ferner vorzugsweise aus einem Gedächtnismetall bestehen. Der Elektrodenkopf weist durch dieses Material bei einer ersten Temperatur eine bestimmte Ursprungsform auf, die für eine Implantation der Elektrodenvorrichtung in einem Blutgefäss geeignet ist. Bei einer zweiten Temperatur, z.B. der Körpertemperatur, erhält der Elektrodenkopf die Form eines Zylinders oder die Konturen eines Zylinders.

Weitere Vorteile und Einzelheiten ergeben sich aus den Unteransprüchen.

Die Erfindung ist nachfolgend anhand von mehreren, in den Zeichnungen dargestellten Ausführungsbeispielen näher erläutert. Es zeigen:

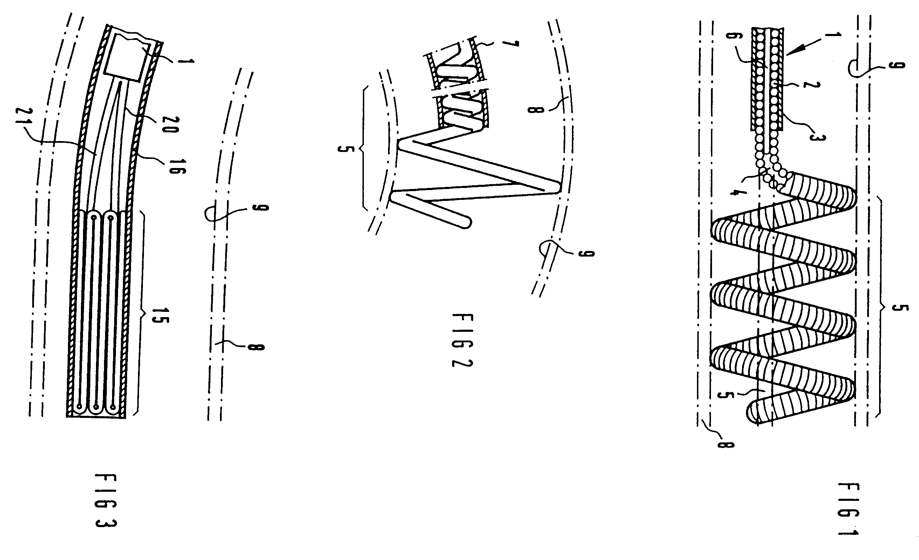

FIG. 1 bis 6 Elektrodenvorrichtungen nach der Erfindung mit Elektrodenkopfen in verschiedenen Ausführungsformen in sowohl passiven als auch in aktiven Lagen.

In der FIG. 1 ist das distale Ende einer Elektrodenvorrichtung für die intravaskulare Plazierung in einem Patienten abgebildet.

Die Elektrodenvorrichtung, die teils in einem Längsschnitt gezeigt ist, umfasst ein Elektrodenkabel 1 bestehend aus einem langgestreckten, wendelförmigen und flexiblen Leiter 2, dessen Aussenseite mit einer Isolierschicht 3 versehen ist und dessen 5 Innenseite einen Kanal 4 bildet. Am distalen Ende des Leiters 2 ist ein Elektrodenkopf 5 zum Defibrillieren des Herzens eines Patienten angebracht. Der Elektrodenkopf 5 ist in diesem Ausführungsbeispiel eine nicht isolierte Verlängerung des Elektrodenkabels 1, so dass der Leiter 2 eine Defibrillierungsoberfläche bildet. Der Leiter 2, der hier der Elektrodenkopf 5 ist, ist in eine schraubenförmig gewickelten Konfiguration geformt und bildet auf diese Weise die Konturen eines hohlen Zylinders. Durch den Aufbau dieser Elektrodenvorrichtung erstreckt sich der Kanal 4 über die ganze Länge der Elektrodenvorrichtung. Bei einer Implantation der Elektrodenvorrichtung in einem Blutgefäss 8 wird ein Mandrin 6 in den Kanal 4 eingeführt, wobei der Elektrodenkopf 5, wie es durch die strichpunktierte Version dargestellt ist, ausgerichtet wird. Die Elektrodenvorrichtung erhält dadurch einen Aussendurchmesser, der kleiner ist als der Innendurchmesser derjenigen Blutgefässe, die Passiert werden sollen und auch kleiner als das Blutgefäss 8, in dem der Elektrodenkopf 5 appliziert werden soll. Wenn der implantierende Arzt eine geeignete Lage für den Elektrodenkopf 5 gefunden hat, kann der Mandrin 6 herausgezogen werden, wobei der Elektrodenkopf 5 in radialer Richtung expandiert und dadurch wieder seine vorgeformte schraubenformig gewickelte zylindrische Konfiguration einnimmt. Der Elektrodenkopf 5 ist derart geformt, dass er in einer expandierten Lage, d.h. wenn der Elektrodenkopf die Konturen eines Zylinders 5 aufweist, den Durchmesser desjenigen Blutgefässes 8, in dem der Elektrodenkopf appliziert werden soll, überschreitet, wobei der Elektrodenkopf bzw. der Zylinder 5 gegen die umgebende Gefässwand 9 druckfixiert wird. In der fixierten Lage bildet der Zylinder 5 eine im Ganzen verhältnismässig grosse, gegen die umgebende Gefässwand anliegende Defibrillierungsoberfläche. Gleichzeitig hat die Form des Zylinders 5 den Vorteil, dass das Blut im Gefäss 8 im Grossen und Ganzen frei durch das Innere des Zylinders 5 strömen kann. Die Gefahr, dass Blutklumpen gebildet werden, wird hierdurch minimiert. Ebenso bietet die Elektrodenvorrichtung die Möglichkeit einer einfachen Repositionierung des Elektrodenkopfes bzw. des Zylinders 5 an, indem der Mandrin 6 wieder in den Kanal 4, eingeschoben wird um den Elektrodenkopf auszurichten.

In der FIG. 2 ist dargestellt, dass der Arzt bei der Implantation statt eines Mandrins ein Einführungskatheter 7 verwenden kann. Das Elektrodenkabel 1 mit dem ausgerichteten Elektrodenkopf 5 wird in dem Einführungskatheter 7 appliziert, wobei der Einführungskatheter 7 ausreichend steif ist, um den Elektrodenkopf 5 während der Implantation in einer ausgerichteten Lage zu halten. In dieser FIG. ist auch gezeigt, dass der Elektrodenkopf 5 in Form einer Miniwendel in dem Einführungskatheter appliziert werden kann. Wenn der Elektrodenkopf 5 in eine gewünschte Lage eingeführt ist, kann der Einführungskatheter 7 zurückgezogen werden, so dass der Elektrodenkopf 5 wieder seine vorgeformte Konfiguration einnimmt und dadurch einen Druck gegen die Innenwand 9 des Blutgefässes 8 ausübt, was zum Teil in der FIG. 2 gezeigt ist.

In der FIG. 3 ist ein weiteres Beispiel eines Elektrodenkopfes 15, der eine derartige Form aufweist, dass er in einem Einführungskatheter 16 applizierbar ist, gezeigt. Der Einführungskatheter ist ausreichend stark, um den Elektrodenkopf 15 während der Implantation zusammengedrückt zu halten. Wenn der Elektrodenkopf 15 in eine gewünschte Lage im Blutgefäss 8 eingeführt worden ist, kann der Einführungskatheter 16 zurückgezogen werden, so dass der Elektrodenkopf 15 in radialer Richtung expandiert und in einer expandierten Lage die Konturen eines Hohlzylinders bildet. Der Zylinder 15 besteht aus mindestens einem drahtförmigen Leiter 17, der zickzackförmig entlang der Längsachse des Zylinders 15 verläuft. Um deutlich den Aufbau des Zylinders darzustellen, ist dessen Mantelfläche nur mit einer kleineren Anzahl zickzackförmiger Abschnitte versehen. Der Zylinder 15 kann selbstverständlich mit einer grösseren Anzahl solcher zickzackförmiger Abschnitte, die sich zwischen den gestrichelten Linien 18, 19 erstrecken, versehen sein. Damit in dem Zylinder 15 eine gute Stromverteilung erhalten wird, ist das Elektrodenkabel 1 über die Leiter 20, 21 an zwei Stellen am Zylinder 15 angeschlossen.

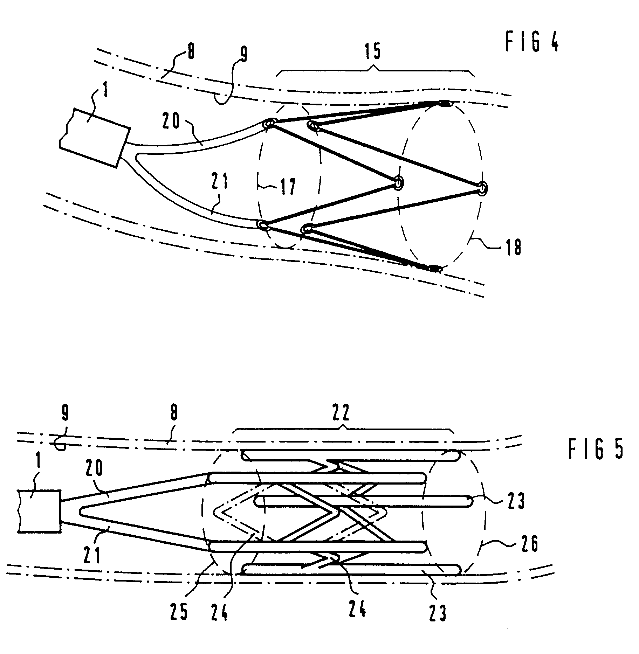

In der FIG. 5 ist ein weiteres Beispiel eines Elektrodenkopfes gezeigt, der derart aufgebaut ist, dass er in einer nicht expandierten Lage in einem Einführungskatheter applizierbar ist. Dieser Elektrodenkopf 22 besteht aus parallel zueinander verlaufenden drahtförmigen Leitern 23, die in einer expandierten Lage entlang der Längsachse des Zylinders 22 verlaufen und die mittels zusammendrückbarer Verbindungselemente 24 miteinander verbunden sind. Die Verbindungselemente 24 sind in dem Ausführungsbeispiel eine Anzahl nach vorne und/oder nach hinten gerichtete, in der Längsrichtung der Leiter 23 verlaufende V-förmige Teile, die, um die Defibrillierungsoberfläche am Zylinder 22 zu vergrössern, leitend sein können. Auch in diesem Ausführungsbeispiel kann der Zylinder 22 mit einer grösseren Anzahl drahtförmiger Leiter 23 versehen werden, die sich zwischen den gestrichelten Linien 25, 26, die die Konturen der Stirnseiten des fiktiven Zylinders 22 zeigen, erstrecken.

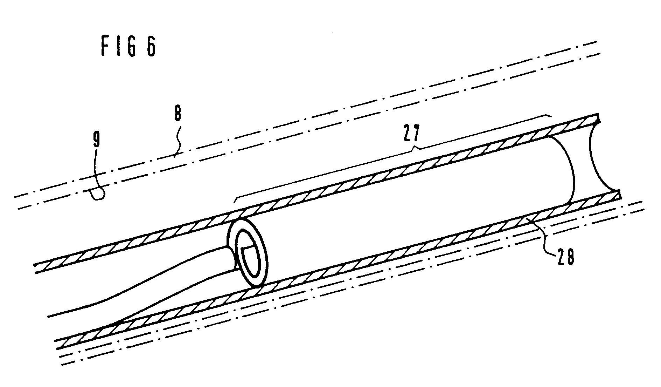

In der FIG. 6 ist ein Elektrodenkopf 27 dargestellt, der aus einer Folie, die in einer nicht expandierten Lage spiralförmig zusammengerollt und mittels eines Einführungskatheters 28 zusammengedrückt ist, besteht.

Wenn der Einführungskatheter 28 vom Elektrodenkopf 27 zurückverschoben wird, wird er zu einem Zylinder expandiert, dessen Mantelfläche gegen die umgebende Blutgefässwand 9 derart anliegt, dass eine Druckfixierung des Zylinders 27 erzielt wird. Die Defibrillierungsoberfläche dieses Zylinders 27 ist extrem gross. Die beschriebenen Elektrodenköpfe bzw. Zylinder 5, 10, 15, 22, 27 können auch aus einem Gedächtnismetall bestehen, was bedeutet, dass die Elektrodenkopfe bei einer ersten Temperatur eine Form aufweisen, die für eine Implantation geeignet sind und bei einer zweiten Temperatur, vorzugsweise im Bereich der Körpertemperatur, die beschriebene zylindrische Form einnehmen. Hilfsmittel für die Implantation wie Mandrine und Einführungskatheter sind in solchen Fallen nicht notwendig.

Die erwähnten Leiter, die in den verschiedenen Ausführungsbeispielen teils als Zylinder und teils als Folie beschrieben worden sind, sind auf der Seite, die gegen die umgebende Gefässwand anliegt, mit einer Isolierschicht versehen, um zu verhindern, dass bei einer Defibrillierung eventuelle Brennwunden entstehen. In einem solchen Fall besteht die gesamte Innenseite des Zylinders bzw. der Folie aus einer Defibrillierungsoberfläche.

Der Elektrodenkopf kann im Rahmen der Erfindung weitere Konfigurationen als die, die in der Anmeldung gezeigt und beschrieben sind, aufweisen. Gemeinsam für solche Elektroden köpfe ist, dass sie in radialer Richtung expandierbar sind und dass sie in einer expandierten Lage mindestens die Konturen eines Hohlkörpers bilden.
¹ *The reference numeral is given as 29 in the German text, but this is clearly a misprint for 27.*

## Patentansprüche

1. Defibrillierungselektrode, insbesondere für die intravaskulare Plazierung in einem Patienten, mit einem flexiblen Elektrodenkabel, das mindestens einen langgestreckten elektrisch isolierten Leiter umfasst und mit einem am distalen Ende des Elektrodenkabels angebrachten Elektrodenkopf, der mindestens eine Defibrillierungsoberfläche zur Übertragung von Defibrillierungsimpulsen an das Herz des Patienten aufweist, wobei der Elektrodenkopf (5,10,15,22,27) derart aufgebaut ist, dass er in radialer Richtung expandierbar ist und in einer expandierten Lage wenigstens die Konturen eines Hohlkörpers (5,10,15,22,27) bildet, wobei der Radius des Hohlkörpers (5,10,15,22,27) den Radius des Blutgefässes (8) derart überschreitet, dass der Hohlkörper (5,10,15,22,27) bei einer Applikation gegen die umgebende Gefässwand (9) druckfixiert wird, **dadurch gekennzeichnet**, dass die nach innen gerichtete Wand des gesamten Hohlkörpers (5,10,15,22,27) die Defibrillierungsoberfläche bildet.

2. Defibrillierungselektrode nach Anspruch 1, **dadurch gekennzeichnet**, dass der Hohlkörper (5) aus einem schraubenförmig gewickelten Leiter (2) besteht.

3. Defibrillierungselektrode nach Anspruch 2, **dadurch gekennzeichnet**, dass der Leiter (2) des Elektrodenkabels (1) und der schraubenförmig gewickelte Leiter (2,13,14) einen Kanal (4) aufweisen, der sich über die gesamte Länge der Defibrillierungselektrode erstreckt und der zum Einführen eines Mandrins (6) vorgesehen ist.

4. Defibrillierungselektrode nach einem der Ansprüche 1 bis 3, **dadurch gekennzeichnet**, dass der Hohlkörper (5,10,15,22,27) die Form eines Zylinders aufweist.

5. Defibrillierungselektrode nach Anspruch 1, **dadurch gekennzeichnet**, dass der Elektrodenkopf (27) aus einer Folie besteht, die in einer nicht expandierten Lage spiralförmig zusammengerollt ist.

6. Defibrillierungselektrode nach Anspruch 4, **dadurch gekennzeichnet**, dass der Zylinder (22) aus parallel zueinander verlaufenden drahtförmigen Leitern (23) besteht, die entlang der Längsachse des Zylinders (22) verlaufen und die mittels zusammendrückbarer Verbindungselemente (24) miteinander verbunden sind.

7. Defibrillierungselektrode nach Anspruch 6, **dadurch gekennzeichnet**, dass die Verbindungselemente (24) nach vorne und/oder nach hinten gerichtete, in der Längsrichtung der Leiter (23) verlaufende V-förmige, vorzugsweise leitende Teile sind.

8. Defibrillierungselektrode nach Anspruch 4, **dadurch gekennzeichnet**, dass der Zylinder (15) aus mindestens einem drahtförmigen Leiter (17) besteht, der zickzackförmig entlang der Längsachse des Zylinders (15) verläuft.

9. Defibrillierungselektrode nach einem der Ansprüche 1 bis 8, **dadurch gekennzeichnet**, dass der Elektrodenkopf (5,10,15,22,27) in einer nicht expandierten Lage in einem Einführungskatheter (7,16,28) applizierbar ist.

10. Defibrillierungselektrode nach einem der Ansprüche 1 bis 9, **dadurch gekennzeichnet**, dass der Elektrodenkopf (5,10,15,22,29) aus einem Gedächtnismetall besteht.

## Claims

1. Defibrillation electrode, particularly intravascular placing in a patient, with a flexible electrode cable which includes at least one elongated electrically insulated conductor and with an electrode head which is mounted on the distal end of the electrode cable and which has at least one defibrillation surface for transmitting defibrillation pulses to the patient's heart, wherein the electrode head (5, 10, 15, 22, 27) is constructed in such a way that it is expandable in the radial direction and in an expanded position forms at least the contours of a hollow body (5, 10, 15, 22, 27), the radius of the hollow body (5, 10, 15, 22, 27) exceeding the radius of the blood vessel (8) in such a way that when the hollow body (5, 10, 15, 22, 27) is applied against the surrounding vessel wall (9) it is fixed by pressure, characterised in that the inwardly directed wall of the entire hollow body (5, 10, 15, 22, 27) forms the defibrillation surface.

2. Defibrillation electrode as claimed in Claim 1, characterised in that the hollow body (5) consists of a spirally coiled conductor (2).

3. Defibrillation electrode as claimed in Claim 2, characterised in that the conductor (2) of the electrode cable (1) and the spirally coiled conductor (2, 13, 14) have a channel (4) which extends over the entire length of the defibrillation electrode and is provided for the introduction of a mandrel (6).

4. Defibrillation electrode as claimed in one of Claims 1 to 3, characterised in that the hollow body (5, 10, 15, 22, 27) has the shape of a cylinder.

5. Defibrillation electrode as claimed in Claim 1, characterised in that the electrode head (27) consists of a film which in the unexpanded state is rolled spirally together.

6. Defibrillation electrode as claimed in Claim 4, characterised in that the cylinder (22) consists of filamentary conductors (23) which extend parallel to one another along the longitudinal axis of the cylinder (22) and are connected to one another by means of compressible connecting elements (24).

7. Defibrillation electrode as claimed in Claim 6, characterised in that the connecting elements (24) are V-shaped, preferably conductive parts which are directed towards the front and/or towards the rear and extend in the longitudinal direction of the conductor (23).

8. Defibrillation electrode as claimed in Claim 4, characterised in that the cylinder (15) consists of at least one filamentary conductor (17) which extends in a zigzag manner along the longitudinal axis of the cylinder (15).

9. Defibrillation electrode as claimed in one of Claims 1 to 8, characterised in that the electrode head (5, 10, 15, 22, 27) can be applied in the unexpanded slate in an insertion catheter (7, 16, 28).

10. Defibrillation electrode as claimed in one of Claims 1 to 9, characterised in that the electrode head (5, 10, 15, 22, 27¹) is made from a memory metal.

## Revendications

1. Electrode de défibrillation, notamment pour une mise en place intravasculaire dans un patient, comprenant un câble d'électrode souple, qui contient au moins un conducteur électriquement isolé s'étendant en longueur, et comprenant une tête d'électrode, qui est montée à l'extrémité distale du câble d'électrode et qui comporte au moins une surface de défibrillation pour transmettre des impulsions de défibrillation au coeur du patient, la tête (5, 10, 15, 22, 27) d'électrode étant conçue de telle sorte qu'elle peut se détendre en direction radiale et forme, dans une position détendue, au moins les contours d'un corps (5, 10, 15, 22, 27) creux, le rayon du corps (5, 10, 15, 22, 27) creux étant supérieur au rayon du vaisseau (8) sanguin de telle sorte que le corps (5, 10, 15, 22, 27) creux est, lors d'une application, fixé par pression contre la paroi (9) entourante du vaisseau, **caractérisée** en ce que la paroi de l'ensemble du corps (5, 10, 15, 22, 27) creux qui est dirigée vers l'intérieur forme la surface de défibrillation.

2. Electrode de défibrillation suivant la revendication 1, **caractérisée** en ce que le corps (5) creux est constitué d'un conducteur (2) enroulé en hélice.

3. Electrode de défibrillation suivant la revendication 2, **caractérisée** en ce que le conducteur (2) du câble (1) d'électrode et le conducteur (2, 13, 14) enroulé en hélice comprennent un canal (4) qui s'étend sur toute la longueur de l'électrode de défibrillation et qui est prévu pour l'introduction d'un mandrin (6).

4. Electrode de défibrillation suivant l'une des revendications 1 à 3, **caractérisée** en ce que le corps (5, 10, 15, 22, 27) creux possède la forme d'un cylindre.

5. Electrode de défibrillation suivant la revendication 1, **caractérisée** en ce que la tête (27) d'électrode est constituée d'une feuille qui est enroulée en spirale dans une position non détendue.

6. Electrode de défibrillation suivant la revendication 4, **caractérisée** en ce que le cylindre (22) est constitué de conducteurs (23) sous forme de fils métalliques s'étendant parallèlement entre eux, qui s'étendent le long de l'axe longitudinal du cylindre (22) et qui sont mutuellement reliés par des éléments (24) de liaison pouvant être réunis par compression.

7. Electrode de défibrillation suivant la revendication 6, **caractérisée** en ce que les éléments (24) de liaison sont des éléments en forme de V, de préférence conducteurs, s'étendant dans la direction longitudinale des conducteurs (23) et dirigés vers l'avant et/ou vers l'arrière.

8. Electrode de défibrillation suivant la revendication 4, **caractérisée** en ce que le cylindre (15) est constitué d'au moins un conducteur (17) sous forme de fil métallique, qui s'étend en zigzag le long de l'axe longitudinal du cylindre (15).

9. Electrode de défibrillation suivant l'une des revendications 1 à 8, **caractérisée** en ce que la tête (5, 10, 15, 22, 27) d'électrode peut, dans une position non détendue, être appliquée dans un cathéter (7, 16, 28) d'introduction.

10. Electrode de défibrillation suivant l'une des revendications 1 à 9, **caractérisée** en ce que la tête (5, 10, 15, 22, 27) d'électrode est réalisée en un métal à effet de mémoire.
